(19) Europäisches Patentamt
European Patent Office
Office européen des brevets

(11) Publication number: **0 369 082 A1**

(12) # EUROPEAN PATENT APPLICATION

(21) Application number: 88402853.1

(22) Date of filing: 14.11.88

(51) Int. Cl.[5]: C07D 311/72

(43) Date of publication of application: 23.05.90 Bulletin 90/21

(84) Designated Contracting States: FR

(71) Applicant: MERRELL DOW PHARMACEUTICALS INC.
2110 East Galbraith Road
Cincinnati Ohio 45215-6300(US)

(72) Inventor: Grisar, J. Martin
Lotissement Wingersbach 7, Rue de Mulhouse
F-67160 Wissembourg(FR)
Inventor: Petty, Margaret
49, Rue de l'Yser
F-67000 Strasbourg(FR)
Inventor: Bolkenius, Frank
Bierkellerstrasse 15B
D-7640 Kehl(DE)

(74) Representative: Gillard, Marie-Louise et al
Cabinet Beau de Loménie 55, Rue d'Amsterdam
F-75008 Paris(FR)

(54) **Cardioprotective tocopherol analogs.**

(57) This invention relates to alkylamino alkylene derivatives of certain 2H-1-benzopyrans, to the intermediates are processes useful for their preparation, to their free-radical scavenger and cellular protective properties and to their end-use application as therapeutic agents.

EP 0 369 082 A1

## CARDIOPROTECTIVE TOCOPHEROL ANALOGS

This invention relates to alkylamino alkylene derivatives of certain 2H-1-benzopyrans, to the intermediates and processes useful for their preparation, to their free-radical scavenger and cellular protective properties and to their end-use application as therapeutic agents.

More specifically this invention relates to alkylamino alkylene derivatives of the formula

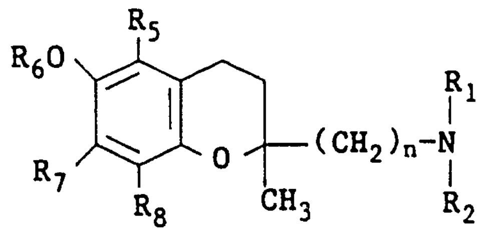

I

and the (R) and (S) enantiomers and racemic mixtures thereof wherein
$R_1$ and $R_2$, each individually is a $C_{1-6}$ alkyl,
$R_5$ is H or $C_{1-6}$ alkyl,
$R_6$ is H or $C_{1-10}$ acyl,
$R_7$ is H or $C_{1-6}$ alkyl,
$R_8$ is H or $C_{1-6}$ alkyl and n is an integer of 1 to 6.

As used herein, the moiety $(CH_2)_n$ of Formula I wherein n is an integer of one to six represents a $C_{1-6}$ straight or branched-chain alkylene including such preferred species as methylene, ethylene, propylene, t-butylene, n-butylene, n-hexylene and isopropylene. The term "$C_{1-6}$ alkyl" includes the straight and branched-chain radicals having up to six carbon atoms with methyl, ethyl, propyl, n-butyl, t butyl, pentyl and hexyl being representative. The term "$C_{1-10}$ acyl", i.e. RCO-, embraces the straight and branched-chain alkylcarbonyl moieties having up to ten carbon atoms including methylcarbonyl, ethylcarbonyl, propylcarbonyl, t-butylcarbonyl and n-hexylcarbonyl as preferred representatives.

In general the compounds of Formula I may be prepared by standard chemical processes and techniques analogously known in the art. In practice, the preparation of the compounds of Formula I conveniently utilizes 3,4-dihydro-2,5,7,8-tetramethyl-2H-1-benzopyran-2-ols as starting materials which, for the most part, are known compounds. In those instances wherein any specific starting material is not known then such compounds may readily be prepared using the standard procedures analogously known in the art as well as by applying such processes as would be reasonably expected to produce the desired starting materials.

The preparation of the 3,4-dihydro-2,5,7,8-tetramethyl-2H-1-benzopyran-2-ols and their conversion to the final products of Formula I is depicted in the following reaction scheme.

REACTION SCHEME A:

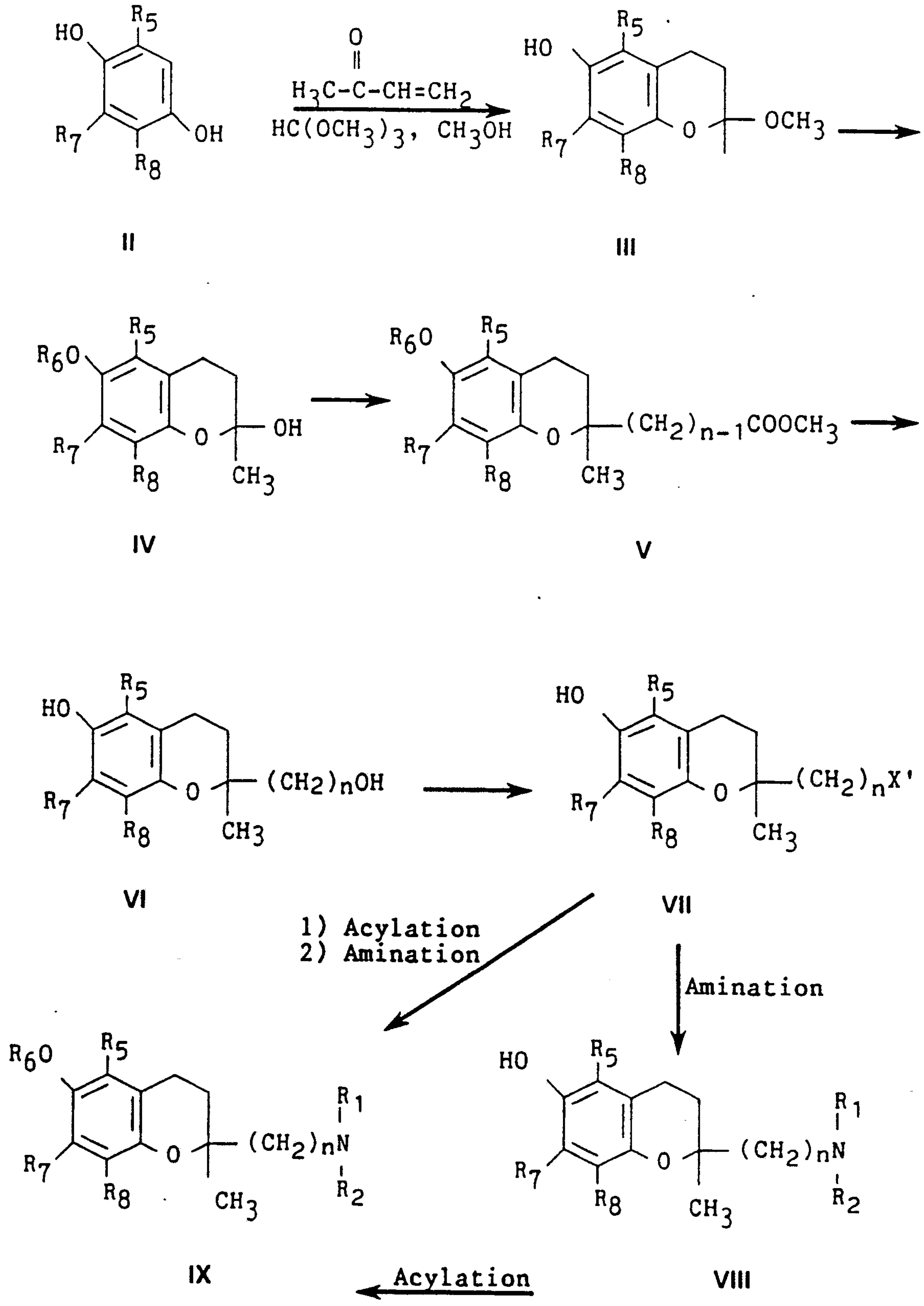


II III IV V VI VII VIII IX

wherein $R_1$, $R_2$, $R_5$, $R_6$, $R_7$ and $R_8$, n and X are as previously defined and X′ is a halide or a tosylate activating moiety for amination.

The reaction of Scheme A entails the condensation of hydroquinones (II) with 3-butene-2-one in the presence of an acid, preferably sulfuric acid, the condensation being effected in methanol and trimethyl orthoformate. The so-produced dihydrobenzopyrans (III) are then sequentially subjected to acylation and

hydrolysis reactions according to standard procedures to yield the hemiketals of Formula (IV). Introduction of the hydroxyalkyl moiety at the 2-position of the compounds of Formula (IV) can be effected by Wittig or Horner type reactions, preferably by reaction of the compounds of Formula (IV) with a trimethylphosphonoester (e.g. trimethylphosphonoacetate) to yield the esters of Formula (V) which are hydrolyzed, and then reduced (preferably with lithium aluminum hydride) to yield the alcohols of Formula (VI). These alcohols may also be formed directly by an acid catalyzed condensation of the hydroquinones (II) with the appropriate vinyl diols of Formulae (IX )and (X).

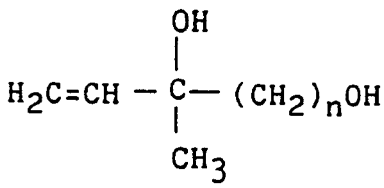

IX

or

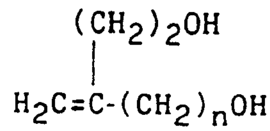

X

n being as defined above.

Prior to amination, the alcohols of Formula (VI) are first activated by converting the 2-position hydroxyalkyl moieties to either their halides or tosylates (i.e., $X'$ is a halide or a p-toluenesulfonyl radical) according to standard conditions such as for example reaction of the alcohols with bromotriphenyl phosphonium bromide ($Ø_3PBr^+Br^-$) obtained by reaction of triphenylphosphine with bromine in dichloromethane. The resulting activated halides (or tosylates) (VII) may be converted to the desired dialkylamino derivatives either before or after acylation of the 6-OH moiety. Standard procedures such as the reaction of the activated moiety with the appropriate dialkylamine, i.e., contracting equimolar quantities of the reactants at temperatures of about 30 °C to 90 °C with stirring in an inert solvent, preferably dimethylformamide, may be used to obtain the dialkylamino derivative, and standard acylation procedures such as reaction of the 6-OH moiety with an acyl halide, carboxylic anhydride or carboxylic acid produce the desired alkylcarbonyloxy moiety at the 6-position.

Further, as there is an asymmetric carbon atom at the 2-position, the compounds may occur as either the R-or the S-enantiomers, or mixtures thereof. The preparation of the individual enantiomeric form may be effected by resolving the acids of Formula (V) by standard and conventional means such as, for example, via the use of diastereomeric salts with optically active amines, or alternatively, by resolving the alcohols (VII) as esters with optically active acids, e.g. L-2,4-$MeClC_6H_3CHMeCOOH$ (Me representing methyl).

The following examples will serve to illustrate the techniques and processes described herein.

## EXAMPLE 1

### 3,4-Dihydro-2-(2-bromoethyl)-2,5,7,8-tetramethyl-2H-1-benzopyran-6-ol

To 11.0 g (0.042 m) of triphenylphosphine in 200 ml of dichloromethane is added dropwise a solution of 6.71 g (0.042 m) of bromine in 50 ml of dichloromethane. The solution is stirred for 30 min at room temperature, then 10.0 g (0.04 m) of 3,4-dihydro-6-hydroxy-2,5,7,8-tetramethyl-2H-1-benzopyran-2-ethanol (CAS 79907-48-5) is added. The resulting solution is refluxed for 4 hours, allowed to cool overnight, washed with a solution of 15 g of sodium carbonate in 200 ml of water, dried over anhydrous sodium sulfate, filtered and evaporated. The resulting oil is crystallized from methanol to give 9.22 g of 3,4-dihydro-2-(2-bromoethyl)-2,5,7,8-tetramethyl-2H-1-benzopyran-6-ol.

The optically active enantiomers are obtained by substituting racemic 3,4-dihydro-6-hydroxy-2,5,7,8-tetramethyl-2H-1-benzopyran-3-ethanol with enantiomer R-(CAS 94425-68-0) or S-(CAS-94425-67-9) and by following the procedures of this example for each individual isomer.

## EXAMPLE 2

3,4-Dihydro-2-(2-bromoethyl)-2.5,7,8-tetramethyl-2H-1-benzopyran-6-yl acetate

To a solution of 9.22 g (0.029 m) of 3,4-dihydro-2-(2-bromoethyl)-2,5,7,8-tetramethyl-2H-1-benzopyran-6-ol in 60 ml of lutidine is added 30 ml of acetic anhydride. The resulting solution is stirred at room temperature overnight. Water (30 ml) is added and some ice to keep the temperature around 30 °C, the mixture is stirred for 30 min, more water and ice are added, the resulting precipitate is collected, washed with water and dried over phosphorus pentoxide under reduced pressure to give 10.0 g of powder. Recrystallization from a mixture of ethyl ether and pentane gives 9.41 g of 3,4-dihydro-2-(2-bromoethyl)-2,5,7,8-tetramethyl-2H-1-benzopyran-6-yl acetate, m.p. 102-103 °C.

EXAMPLE 3

3,4-Dihydro-2-(2-dimethylaminoethyl)-2,5,7,8-tetramethyl-2H-benzopyran-6-yl acetate

A mixture of 3.55 g (0.01 m) of 3,4-dihydro-2-(2-bromoethyl)-2,5,7,8-tetramethyl-2H-benzopyran-6-yl acetate and 2.0 g of liquid dimethylamine in 50 ml of dimethylformamide is stirred at room temperature for 40 hours. Water is added and the product is extracted with ethyl acetate and ethyl ether. The extract is washed with water, dried over anhydrous sodium sulfate, filtered and evaporated. The resulting oil crystallizes from a mixture of ethyl ether and pentane to give 2.05 g of 3,4-dihydro-2-(2 dimethylaminoethyl)-2,5,7,8-tetramethyl-2H-benzopyran-6-yl acetate as the free base.

EXAMPLE 4

3,4-Dihydro-2-(2-dimethylaminoethyl)-2,7,8-trimethyl-2H-1-benzopyran-6-ol

Following the procedure described in Example 3, but using-3,4-dihydro-6-hydroxy-2,7,8-trimethyl-2H-1-benzopyran-2-ethanol (CAS 93600-70-5) as starting material, the title compound is obtained.

EXAMPLE 5

3,4-Dihydro-2-(2-dimethylaminoethyl)-2,5,8-trimethyl-2H-1-benzopyran-6-ol

Following the procedure described in Example 3, but using 3,4-dihydro-6-hydroxy-2,5,8-trimethyl-2H-1-benzopyran-2-ethanol (CAS 93600-69-2) as starting material, the title compound is obtained.

EXAMPLE 6

3,4-Dihydro-2-(2-dimethylaminoethyl)-2,5,7-trimethyl-2H-1-benzopyran-6-ol

Following the procedure described in Example 3, but using 3,4-dihydro-6-hydroxy-2,5,7-trimethyl-2H-1-benzopyran-2-ethanol (CAS 93600-68-1) as starting material, the title compound is obtained.

EXAMPLE 7

3,4-Dihydro-2(2-dimethylaminoethyl)-2,5,7,8-tetramethyl-6-(1,1-dimethyl-ethylcarbonyloxy)-2H-1-benzopyran

Following the procedure described in Example 2, but substituting acetic anhydride by an equimolar amount of pivaloyl chloride, 3,4-dihydro-2-(2-bromoethyl)-2,5,7,8-tetramethyl-2H-1-benzopyranyl α,α-dimethylpropionate is obtained, which is then converted to the title compound by the procedure described in Example 3.

## EXAMPLE 8

3,4-Dihydro-3-(3-dimethylaminopropyl)-2,5,7,8-tetramethyl-2H-1-benzopyran-6-ol

Following the procedure described in Example 3, but using 3,4-dihydro-6-hydroxy-2,5,7,8-tetramethyl-2H-1-benzopyran-2-propanol (CAS 104568-57-2) as starting material, the title compound is obtained.

Having described the scope of the compounds of this invention as well as the generic and specific methods for preparing said compounds, the following information describes the utility, and the methods therefor, of the compounds of this invention.

When the blood supply to parts of the heart muscle is blocked, a myocardial infarct (heart attack) results and the deprived muscle tissue dies with the result of permanent heart damage. If the blood supply can be reestablished within hours after infarction, the heart muscle tissue remains viable and permanent damage can be reduced. This can be accomplished by surgical as well as pharmacologic (thrombolysis) procedures and these processes are known as reperfusion.

Reperfusion is now widely and successfully applied and it has been claimed that fatalities due to myocardial infarction can be reduced by 20-30%. However, reperfusion also poses problems. Oxygen-deprived (ischemic) tissue finds itself in an abnormal state and is vulnerable when suddenly exposed to oxygen-rich blood. This has been termed the "oxygen paradox" and leads to reperfusion damage in the form of cell death. It has been postulated that this damage is due to oxygen-derived free radicals and, in particular, to the superoxide radical, $O_2^-$. Evidence for this hypothesis has been obtained in animal experiments. B.R. Lucchesi and coworkers showed that the enzyme superoxide dismutase, as well as the free radical scavenger N-(mercaptopropionyl)glycine reduce canine myocardial reperfusion injury (Cir. Res., 1984, 54, 277-285; J. Cardiovasc. Pharmacol., 1986, 8, 978-88; Fed. Proc., 1987, 46, 2413-21).

Vitamin E, i.e., α-tocopherol, a well known compound of the formula

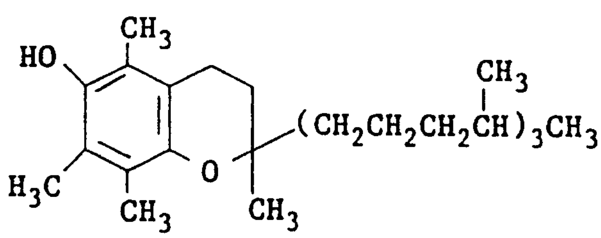

is a natural anti-oxidant that reacts with oxygen-derived free radicals as well as hydrogen peroxide. It has been shown that it is intercalated in lipid membranes and that its biological function is to protect biomembranes against oxidative attack. The anti-oxidant 3,4-dihydro-2,5,7,8-tetramethyl-2H-2-benzopyran-6-ol moiety of α-tocopherol is constantly regenerated by the ubiquitous cytosolic redox systems and for all practical purposes is a permanent membrane constituent that is constantly regenerated.

The compounds of this invention also possess a related or similar 3,4-dihydroxy-2,5,7,8-tetraalkyl-2H-1-benzopyran-2-yl moiety, but the 2-position lipophylic moiety of the α-tocopherol molecule, which is thought to be responsible for its ubiquitous incorporation into biomembranes, is replaced with a hydrophylic moiety to impart a greater affinity for cardiac tissue. Thus, the compounds of this invention are also useful as pharmacologic antioxidants and free radical scavengers and, in particular, as scavengers of superoxide anion radical $O_2^-$. They can be therapeutically employed where reperfusion damage due to oxygen-derived free radicals and hydrogen peroxide causes cell death in tissues. This situation arises when total or partial blockade of blood supply to tissues is removed, either spontaneously (transient ischemia) or by pharmacologic or surgical intervention (thrombolysis, angioplasty, by-pass, organ transplant and the like).

Tissues subjected to transient ischemia or reperfusion in various disease states, or by their medical treatment, are those of heart, lung, kidney, pancreas and brain. In particular, the now rapidly increasing practice of pharmacologic thrombolysis, also known as reperfusion, after coronary infarct and stroke, will benefit by prior or concomitant administration of a free radical scavenger such as the compounds of this invention. Similarly, surgical interventions, such as percutaneous transluminal coronary angioplasty, where a dilating balloon is used to increase the luminal diameter in severely occluded atherosclerotic vessels, coronary by-pass operations, and organ transplant surgery create conditions where reperfusion damage due to oxygen-derived radicals takes place and can be reduced by scavengers. Transient ischemia is one of the causative factors that lead to angina pectoris, and thus the compounds of this invention are also useful as antianginal agents.

The process of inflammation is also known to involve the release of superoxide radicals from phagocytic cells which cause some of the symptoms of rheumatoid arthritis and a free radical scavenger, such as the compounds of this invention, is also useful in the treatment of this disease. The compounds may also be useful in the treatment of cancers and of aging since oxygen-derived free radicals have been identified among causative factors. For reviews, see B. Halliwell and C. Gutteridge, Biochem. J., 1984, 219, 1-14; TINS 1985, 22-6.

*In vitro* and *in vivo* activity for the compounds of this invention may be demonstrated by the use of standard assays which demonstrate the free radical scavenging property, affinity for cardiac tissue and cardioprotective properties.

The following procedure demonstrates the cardioprotective effects of compounds of this invention.

3,4-Dihydro-2-(2-dimethylaminoethyl)-2,5,7,8-tetramethyl-2H-1-benzopyran-6-yl acetate hydrochloride was evaluated in ligation-induced infarcted and reperfused rats as follows. One of two groups of rats was infused intravenously with a solution of test compound in saline at a rate of 2.3 ml/h (10 mg/kg/h). The control group was infused with saline at the same rate. After 10 min of drug, coronary arteries were ligated surgically for 60 min, ligation was loosened to allow reperfusion for 30 min. The ligation was retied and a dye (Evans Blue) was injected. The animals were sacrificied and the heart ventricals were removed and weighed. The unstained tissue was dissected and weighed; this represents the "area at risk", i.e. the area that was deprived of blood supply by ligation. To determine the infarcted area, the tissue was incubated with 2,3,5-triphenyltetrazolium chloride. Infarcted tissue became light colored and was dissected and weighed. Thus, for each rat that survived the ligation a measurement of "area at risk" and of "infarcted area" was obtained and the ratio was calculated as shown in the following tables.

TABLE I

| Control Group | | | | |
|---|---|---|---|---|
| Rat No. | Total ventr. wt.,g. | Tissue at risk mg | Infarcted tissue mg | ratio % |
| 1 | 1.203 | 410 | 403 | 98.3 |
| 2 | 1.521 | 459 | 401 | 87.4 |
| 3 | 1.090 | 249 | 245 | 98.4 |
| 4 | 1.142 | 180 | 154 | 85 |
| 5 | 0.973 | 327 | 78 | 24 |
| 6 | 1.054 | 125 | 113 | 90 |
| 7 | 0.784 | 465 | 321 | 89 |
| Average | | | | 78.9 |
| Standard deviation | | | | 26.2 |

TABLE II

| Treated Group | | | | |
|---|---|---|---|---|
| Rat No. | Total ventr. wt.,g. | Tissue at risk mg | Infarcted tissue mg | ratio % |
| 1 | 1.193 | 478 | 337 | 70.1 |
| 2 | 1.046 | 200 | 80 | 40.3 |
| 3 | 1.383 | 513 | 51 | 10 |
| 4 | 1.078 | 416 | 346 | 83.2 |
| 5 | 1.0 | 457 | 18 | 4 |
| 6 | 0.95 | 411 | 294 | 71.6 |
| 7 | 0.830 | 292 | 51 | 17.5 |
| 8 | 0.895 | 487 | 34 | 7.0 |
| Average | | | | 38.0 |
| Standard deviation | | | | 32.9 |

It can be seen that the untreated group gave a ratio of 78.9% and the treated group gave a ratio of 38.0%, i.e., treatment resulted in 48.2% reduction of infarction. The result is statistically significant (ANOVA) at $p < 0.02$.

Most preferably, the compounds are administered intravenously particularly under crisis situations wherein it is essential that the therapeutic agent be gotten to its site of action as quickly as possible, such as in those emergency conditions caused by coronary infarction, stroke and surgical interventions, conditions which can cause severe reperfusion damage.

The compounds of this invention can be utilized both prophylactically and therapeutically. The amount of active ingredient for therapeutic administration can vary over a wide range and is dependent upon such factors as the species of mammal to be treated, its age, health, sex, weight, nature and the severity of the condition being treated. Generally, a therapeutically effective amount of the active ingredient to be administered will range from about 0.1 mg/kg to 50 mg/kg of body weight per day. For prophylactic administration, corresponding lower doses can be utilized.

The compounds of this invention also can be orally administered, preferably using more active ingredient per day than when parenterally administered, preferably taking divided doses 3 to 4 times per day. Preferably, enteral administration in post "crisis" situations, particularly after release from hospitalized conditions. The compounds can be used in standard dosage unit forms such as tablets, capsules, dragees, lozenges, elixirs, emulsions, suspensions, and in cases wherein topical application is preferred by suppository or sub-lingual administration. Tablets and capsules containing from 100 to 400 mg of active ingredient are preferred modes of enteral administration. Of course, in the treatment of inflammation the preferred method of administration is by depot injection directly to the situs of the inflammation area with follow-up enteral means of administration.

In preparing solid dose forms such as tablets, the active ingredient is generally blended with conventional pharmaceutical carriers or excipients such as gelatin, various starches, lactose, calcium phosphate or powdered sugar. The term pharmaceutical carrier as used herein also includes lubricants employed to improve the flow of tablet granulations and which prevent adhesion of tablet material to the surfaces of tablet dies and punches. Suitable lubricants include, for example, talc stearic acid, calcium stearate, magnesium stearate and zinc stearate. Also included within the definition of a pharmaceutical carrier as used herein, and disintegrating agents added to assist the breakup and dissolution of tablets following administration, as well as coloring and/or flavoring agents to enhance the aesthetic qualities of the tablets and make them more acceptable to the patient.

Suitable liquid excipients for the preparation of liquid dosage unit forms include water and alcohols such as ethanol, benzyl alcohol and the polyethylene glycols, either with or without the addition of a surfactant. In general, the preferred liquid excipients, particularly for injectable preparations, include water, physiological and saline solutions, dextrose and glycol solutions such as an aqueous propylene glycol or polyethylene

glycol solutions. In order to minimize or eliminate irritation at the site of injection, such compositions may contain a non-ionic surfactant having a hydrophile-lipophile balance (HLB) of from about 12 to about 17. The quantity of surfactant in such formulations ranges from about 5 to 15% by weight. The surfactant can be a single component having the above-identified HLB, or a mixture of two or more components having the desired HLB. Illustrative of surfactants useful in parenteral formulations are the class of polyoxyethylene sorbitan fatty acid esters as, for example, sorbitan monooleate and the high molecular weight adducts of ethylene oxide with a hydrophobic base, formed by the condensation of propylene oxide with propylene glycol. In certain topical and parenteral preparations, various oils can be utilized as carriers or excipients. Illustrative of such oils are mineral oils, glyceride oils such as lard oil, cod liver oil, peanut oil, sesame oil, corn oil and soybean oil. For insoluble compounds, suspending agents may be added as well as agents to control the viscosity, as for example, magnesium aluminum silicate or carboxymethylcellulose. In addition to these excipients, buffers, preservatives and emulsifying agents may also be added.

Of course, as is true in most instances wherein certain classes of chemical compounds have been found to have beneficial therapeutic end-use applications, certain sub-generic groups and certain specific compounds are preferred. In this instance the preferred compounds of Formula I are those wherein $R_5$, $R_7$ and $R_8$ are methyl; wherein $R_6$ is methyl carbonyl, t-butylcarbonyl, ethylcarbonyl, propylcarbonyl, pentylcarbonyl; wherein n is 2 (representing an ethylene moiety) and the substituents attached to the nitrogen atom are methyl. Preferred compounds are those as illustrated in the examples showing the preparation of the compounds of Formula I.

## Claims

1. A compound of the formula

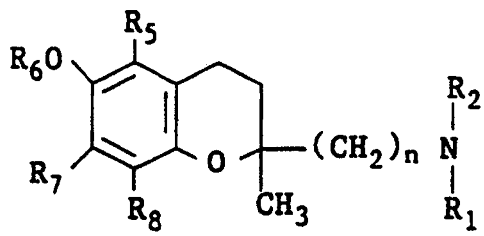

I

and the (R) and (S) enantiomers and racemic mixtures thereof wherein
$R_1$ and $R_2$, each individually is a $C_{1-6}$ alkyl,
$R_5$ is H or $C_{1-6}$ alkyl,
$R_6$ is H or $C_{1-10}$ acyl,
$R_7$ is H or $C_{1-6}$ alkyl,
$R_8$ is H or $C_{1-6}$ alkyl and n is an integer of 1 to 6.

2. Compounds of Claim 1 wherein n is 2.

3. Compounds of Claim 2 wherein $R_5$, $R_7$ and $R_8$ are $CH_3$.

4. Compounds of Claim 3 wherein $R_6$ is hydrogen or methylcarbonyl.

European Patent Office

# EUROPEAN SEARCH REPORT

Application Number

EP 88 40 2853

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | US-A-4 321 270 (J.E. SUNDEEN)<br>* Columns 1,6; column 12, example 5; column 20, example 15 *<br>----- | 1 | C 07 D 311/72 |
| | | | **TECHNICAL FIELDS SEARCHED (Int. Cl.5)** |
| | | | C 07 D 311/00 |

The present search report has been drawn up for all claims

| Place of search | Date of completion of the search | Examiner |
|---|---|---|
| THE HAGUE | 04-07-1989 | FRANCOIS J.C.L. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)